(19) Europäisches Patentamt
European Patent Office
Office européen des brevets

(11) EP 0 621 283 B1

(12) EUROPÄISCHE PATENTSCHRIFT

(45) Veröffentlichungstag und Bekanntmachung des Hinweises auf die Patenterteilung:
**14.01.1998 Patentblatt 1998/03**

(51) Int Cl.[6]: **C07H 15/08**, A61K 7/06, A61K 7/48, C08G 65/08

(21) Anmeldenummer: **94105742.4**

(22) Anmeldetag: **14.04.1994**

(54) **Fettsäureester von Methylglucosid-Derivaten**

Fatty acid esters of methylglucoside derivatives

Esters d'acides gras de dérivés de méthylglucoside

(84) Benannte Vertragsstaaten:
**DE ES FR GB IT**

(30) Priorität: **22.04.1993 DE 4313117**

(43) Veröffentlichungstag der Anmeldung:
**26.10.1994 Patentblatt 1994/43**

(73) Patentinhaber: **Th. Goldschmidt AG**
**45127 Essen (DE)**

(72) Erfinder:
- **Desai, Natvarlei, Dr. rer. nat.**
**D-46537 Dinslaken (DE)**
- **Wisotzki, Klaus Dieter, Dr. rer. nat.**
**D-47475 Kamp-Lintfort (DE)**

(56) Entgegenhaltungen:
**EP-A- 0 415 636** **EP-A- 0 432 646**
**EP-A- 0 507 004**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 621 283 B1

Printed by Jouve, 75001 PARIS (FR)

## Beschreibung

Gegenstand der Erfindung sind Fettsäureester von Methylglucosid-Derivaten, ein Verfahren zu ihrer Herstellung und eine Verwendung.

Glucose- und Saccharose-Derivate können aus nachwachsenden Rohstoffen gewonnen werden. Daher hat das Interesse am Einsatz dieser Chemikalien weltweit zugenommen. Dies hängt damit zusammen, daß die Ausgangsmaterialien aufgrund der landwirtschaftlichen Überproduktion preisgünstig und in unbeschränkter Menge zugänglich sind.

Durch Methylierung von Glucose wird Methylglucosid hergestellt, welches eine gute thermische Stabilität besitzt im Vergleich zur Saccharose. Das Methylglucosid besitzt eine Alpha- und eine Beta-Form. Weiterhin kann auch Stärke und Cellulose für die Produktion von Methylglucosid eingesetzt werden.

Bei der Herstellung von Fettsäureestern von Methylglucosid-Derivaten treten nach den bisher gängigen Verfahren des Standes der Technik erhebliche Nachteile dadurch auf, daß die Kohlenhydrate thermisch instabil sind. Daher kommt es häufig bei derartigen Reaktionen zu einer Karamelisierung und damit Braunfärbung der Produkte, so daß eine Anwendung im kosmetischen Bereich nicht mehr in Frage kommt. Weiterhin ist eine aufwendige Reinigung dieser Produkte notwendig, die den gesamten Prozeß unwirtschaftlicher macht. Ein weiteres Problem ist die mangelhafte Löslichkeit der Substanz in nichttoxischen Lösungsmitteln, die auch im Kosmetikbereich Anwendung finden können.

F.H. Otey et al. in J. Am. Oil Chem. Soc., Vol. 38, Seite 517ff, Oktober 1961, beschreiben die Herstellung von Fettsäureestern des Methylglucosidethers. Dabei wird so vorgegangen, daß zunächst das Methylglucosid bei Temperaturen um 160 bis 175°C mit Ethylenoxid in Mengen von 5 bis 20 Mol ethoxyliert wird. Die Produkte sind bernsteinfarben und besitzen Viskositäten von 21.000 bis 800 cp. Die Produkte werden anschließend mit Natriumhydroxid und Xylol als Lösungsmittel verestert. Danach erfolgt eine Reinigung des erhaltenen Produktes, um Spuren von Xylol aus dem Reaktionsprodukt zu entfernen.

US 4,687,843 beschreibt veresterte mit 5 bis 50 Mol Propylenoxid propoxylierte Methylglucosid-Verbindungen, die erhalten werden durch Reaktion eines propoxylierten Methylglucosids mit einer Fettsäure bei erhöhter Temperatur in Gegenwart eines sauren Katalysators. Die Produkte werden als Feuchtigkeitsmittel in Hautschutzformulierungen verwendet. Diese Produkte sind lediglich Emulgatoren, die wegen ihrer geringen Wasserlöslichkeit nicht für wäßrige Zubereitungen wie Shampoos und Schaumbäder geeignet sind. Weiterhin weisen diese Produkte keine viskositätsverbessernden Eigenschaften auf.

US 4,268,498 beschreibt die Verwendung von Polyoxyethylen (17 bis 23) Glukosefettsäureestern als Basismaterial in kosmetischen Stiften.

EP 0 432 646 A2 beschreibt Alkoxy-alkylglucoside, die mit quaternären Ethergruppen verknüpft sind. Es handelt sich um kationenaktive stickstoffhaltige Verbindungen, die demzufolge Nitrosamine bilden können.

US 5,059,443 beschreibt niedrig ethoxylierte Alkylglucoside, die in niedrigkalorischen fetthaltigen Lebensmittelzubereitungen eingesetzt werden sollen. Sie sind schlecht wasserlöslich und weisen keine viskositätsverbessernden Eigenschaften auf.

Das technische Problem der Erfindung war es, neue stickstofffreie, nichtionogene Fettsäureester von Methylglucosid-Derivaten zur Verfügung zu stellen, die in erheblich reinerer Form vorliegen als die aus dem Stand der Technik bekannten anderen Methylglucosid-Derivate und somit besser zur Verwendung in kosmetischen Zubereitungen wie Shampoos und Schaumbädern geeignet sind.

Das technische Problem wird gelöst durch Fettsäureester von Methylglucosid-derivaten der Formel I

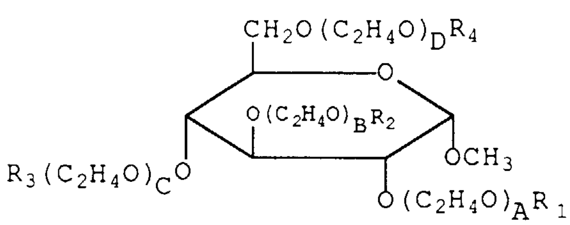

worin A, B, C, D = 21 bis 75 ist, $R_1$, $R_2$, $R_3$, $R_4$ = H oder

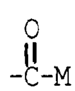

ist, wobei $R_1$ - $R_4$ nicht gleichzeitig H ist und M = $C_{11}$ - $C_{18}$-alkenyl oder $C_{11}$ - $C_{18}$-alkyl ist.

In einer bevorzugten Ausführungsform ist $R_1$, $R_4$ =

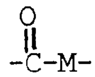

und $R_2$, $R_3$ = H oder $R_1$ =

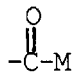

und $R_2$, $R_3$, $R_4$ = H oder $R_4$ =

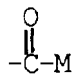

und $R_1$, $R_2$, $R_3$ = H. In einer besonders bevorzugten Ausführungsform ist M = $C_{17}$ alkenyl.

Die erfindungsgemäßen Fettsäureester werden hergestellt durch Ethoxylierung von Methylglucosid mit 84 bis 300 Mol, vorzugsweise 120 Mol Ethylenoxid. Anschließend wird mit 2 bis 4 Mol, vorzugsweise 1,8 bis 3,5 Mol einer $C_{11}$ bis $C_{18}$ gesättigten oder ungesättigten Fettsäure lösungsmittelfrei mit sauren Katalysatoren verestert.

Als Fettsäuren werden bevorzugt Ölsäure, Stearinsäure, Linolsäure, Linolensäure, Laurinsäure oder ein Gemisch derselben verwendet.

Als saure Katalysatoren können Schwefelsäure, Salzsäure, Phosphorsäure, p-Toluolsulfonsäure, Methansulfonsäure, Ethansulfonsäure, Bortrifluorid sowie weitere organische bzw. anorganische Säuren eingesetzt werden.

Die Veresterung erfolgt ohne Lösungsmittel bei Temperaturen zwischen 110°C und 150°C unter Normaldruck und vorzugsweise im Vakuum bei 50 bis 150 mbar und 80°C bis 100°C.

Durch das erfindungsgemäße Verfahren werden Fettsäureester erhalten, die als helle Produkte anfallen. Es kann keine Zersetzung oder Karamelisierung festgestellt werden.

Es wurde ferner überraschenderweise festgestellt, daß die erfindungsgemäß hergestellten Fettsäureester, im Gegensatz zu den erst veresterten und im zweiten Reaktionsschritt ethoxylierten Fettsäureestern, eine erhebliche Viskositätsverbesserung in unterschiedlichen Tensidsystemen aufweisen.

Die erfindungsgemäß hergestellten Produkte erlauben daher mit relativ niedrigen Dosierungen optimale Viskositätseinstellungen der Bade- und Shampoopräparate.

Tabelle 1

| Tensidmischungen | Gew.-% | Vergleichsbeispiel (Viskosität mPas) | Bsp. 1 | Bsp. 2 |
|---|---|---|---|---|
| Produkt<br>Natriumlaurylethersulfat (30 %)<br>Cocamidopropylbetain (30 %)<br>Wasser | 2,5<br>9,0<br>3,0<br>85,5 | 18.000 | 24.600 | 25.200 |
| Produkt<br>Dinatriumlaurylethersulfosuccinate (32 %)<br>Wasser | 4,0<br>12,0<br>44,0 | 38.000 | 52.700 | 52.500 |
| Produkt<br>Natriumlaurylethersulfat (30 %)<br>Wasser | 4,0<br>12,0<br>84,0 | 40.800 | 58.900 | 57.700 |
| Produkt<br>Cocoamphocarboxyglycinate (40 %) | 4,0<br>12,0 | 17.680 | 25.500 | 26.400 |

Tabelle 1 (fortgesetzt)

| Tensidmischungen | Gew.-% | Vergleichsbeispiel (Viskosität mPas) | Bsp. 1 | Bsp. 2 |
|---|---|---|---|---|
| Wasser | 84,0 | | | |

Alle Viskositätsmessungen wurden bei 25°C durchgeführt.

Die erfindungsgemäßen Fettsäureester werden als Verdicker für tensidhaltige Lösungen in kosmetischen Zubereitungen verwendet.

Die erfindungsgemäß hergestellten Fettsäureester wirken jedoch nicht nur verdickend in tensidhaltigen Zubereitungen, sondern weisen zusätzlich eine sehr gute feuchtigkeitsspendende Wirkung auf und reduzieren die Irritationswerte gängiger Inhaltsstoffe wie zum Beispiel anionischer und nichtionischer Tenside in Shampoo- und Badepräparaten.

Die folgenden Beispiele sollen die Erfindung näher erläutern:

## BEISPIELE

### Vergleichsbeispiel

In einem 1 l Dreihalskolben mit vakuumdichtem Rührwerk, regelbarer Temperaturmeßeinrichtung und absteigendem Kühler mit kühlbarer Vorlage werden 508,5 g (1,8 Mol) Ölsäure mit 194,1 g (1,0 Mol) Methylglucosid und 1,5 g Kaliumcarbonat bei 180°C und $10^4$ Pa (100 mbar) Vakuum innerhalb von drei Stunden verestert. Es werden hierbei 662,5g $\alpha$-D-Methylglucosiddioleat als hellgelbes Öl mit folgenden Kenndaten erhalten:

| | |
|---|---|
| Verseifungszahl | 137 |
| Säurezahl | 7 |
| pH-Wert (5 % MeOH/Wasser) | 7,5. |

### Ethoxylierung

500 g (0,967 Mol) $\alpha$-D-Methylglucosiddioleat werden mit 2 g NaOH versetzt und mit 5105,75 g (116,04 Mol) Ethylenoxyd bei 140°C nach bekannten Verfahren ethoxyliert. Der ethoxylierte $\alpha$-D-Methylglucosiddioleatester weist folgende Kenndaten auf:

| | |
|---|---|
| Verseifungszahl | 14 |
| Säurezahl | 1 |
| Wassergehalt | 0,19 % |
| Farbe (Gadner) | 2. |

### Beispiel 1

In einem 1 l Dreihalskolben mit vakuumdichtem Rührwerk, regelbarer Temperaturmeßeinrichtung und absteigendem Kühler mit külbarer Vorlage werden 547,4 g (0,1 Mol) Polyoxyethylen - 120 Methylglucosid, 50,85 g (0,18 Mol) Ölsäure und 2,0 g Schwefelsäure unter vermindertem Druck von $10^4$ Pa (100 mbar) bei 140°C innerhalb von 5 Stunden umgesetzt. Nach Ablauf der Reaktionszeit wird das Produkt mit 5 g (50 %ig) NaOH neutralisiert, wobei 580 g eines hellgelben Reaktionsproduktes mit folgenden Kennzahlen erhalten werden:

| | |
|---|---|
| Verseifungszahl | 17 |
| Säurezahl | 1 |
| Hydroxylzahl | 22 |
| pH-Wert (5 % MeOH/Wasser) | 6,5. |

### Beispiel 2

56,5 g (0,2 Mol) Ölsäure werden mit 547,4 g (0,1 Mol) Polyoxyethylen - 120 Methylglucosid und 2,0 g p-Toluolsulfonsäure nach Beispiel 1 in 5 Stunden umgesetzt. Hierbei werden 583 g eines hellgelben Reaktionsproduktes mit folgenden Kennzahlen erhalten:

| | |
|---|---|
| Verseifungszahl | 19 |
| Säurezahl | 2 |
| Hydroxylzahl | 20 |
| pH-Wert (5 % MeOH/Wasser) | 6,2. |

Das Viskositätsverhalten der nach den Beispielen 1 und 2 hergestellten Produkte wurde in unterschiedlichen Tensidmischungen geprüft. Diese Ergebnisse, sowie die Viskositäten des Vergleichsbeispieles, sind in der Tabelle 1 dargestellt.

Beispiele 3 und 4

| **Babyshampoo** | | |
|---|---|---|
| | Gew.-% | |
| | 3 | 4 |
| Natriumlaurylethersulfat (28 %ig) | 35,0 | 35,0 |
| Cocoamphocarboxyglycinat (40 %ig) | 8,0 | -- |
| Cocamidopropylbetain (30 %ig) | -- | 7,0 |
| Produkt nach Beispiel 1 | 2,5 | -- |
| Produkt nach Beispiel 2 | -- | 2,5 |
| Konservierungsmittel | 0,1 | 0,1 |
| Wasser | 54,4 | 55,4 |
| | 100,0 | 100,0 |

Beispiel 5

| **Shampoo (als Gel)** | |
|---|---|
| | Gew.-% |
| Natriumlaurylethersulfat (30 %ig) | 36,0 |
| Cocamidopropylbetain (30 %ig) | 20,0 |
| GRILLOCAM® E 20 (Methyl Gluceth-20) | 4,0 |
| Produkt nach Beispiel 2 | 3,5 |
| Konservierungsmittel | 0,1 |
| Wasser | 36,4 |
| | 100,0 |

**Patentansprüche**

**1.** Fettsäureester von Methylglucosid-Derivaten der Formel I

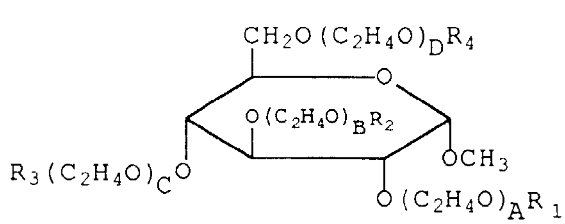

worin

A, B, C, D = 21 - 75 ist
$R_1$, $R_2$, $R_3$, $R_4$ = H oder

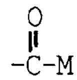

ist,

wobei

$R_1$ - $R_4$ nicht gleichzeitig H ist und
M = $C_{11}$ - $C_{18}$-alkenyl oder $C_{11}$ - $C_{18}$-alkyl ist.

**2.** Fettsäureester nach Anspruch 1, dadurch gekennzeichnet, daß
$R_1$, $R_4$ =

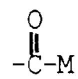

und $R_2$, $R_3$ = H.

**3.** Fettsäureester nach Anspruch 1, dadurch gekennzeichnet, daß
$R_1$ =

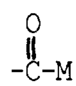

und $R_2$, $R_3$, $R_4$ = H.

**4.** Fettsäureester nach Anspruch 1, dadurch gekennzeichnet, daß
$R_4$ =

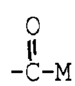

und $R_1$, $R_2$, $R_3$ = H.

**5.** Fettsäureester nach Ansprüchen 1 bis 4, dadurch gekennzeichnet, daß M = $C_{17}$ alkenyl ist.

**6.** Verfahren zur Herstellung des Fettsäureesters nach den Ansprüchen 1 bis 5, dadurch gekennzeichnet, daß

a) ein Methylglucosid mit 84 bis 300 Mol Ethylenoxid ethoxyliert wird und
b) anschließend mit 2 bis 4 Mol einer $C_{11}$ bis $C_{18}$ gesättigten oder ungesättigten Fettsäure lösungsmittelfrei mit einem sauer wirkenden Katalysator verestert wird.

**7.** Verfahren nach Anspruch 6, dadurch gekennzeichnet, daß als Fettsäure Ölsäure, Stearinsäure, Linolsäure, Linolensäure, Laurinsäure oder ein Gemisch derselben verwendet wird.

**8.** Verfahren nach den Ansprüchen 6 oder 7, dadurch gekennzeichnet, daß als Katalysator anorganische oder organische Säuren verwendet werden.

**9.** Verfahren nach Anspruch 8, dadurch gekennzeichnet, daß als Katalysator Schwefelsäure, Salzsäure, Phosphorsäure, p-Toluolsulfonsäure, Methansulfonsäure, Ethansulfonsäure, Bortrifluorid verwendet werden.

**10.** Verfahren nach den Ansprüchen 6 bis 9, dadurch gekennzeichnet, daß bei Temperaturen zwischen 110°C und 150°C ohne Lösungsmittel umgesetzt wird.

**11.** Verwendung der Fettsäureester nach Anspruch 1 bis 5 als Verdicker für tensidhaltige Lösungen in kosmetischen Zubereitungen.

**Claims**

**1.** Fatty acid esters of methylglucoside derivatives of the formula I

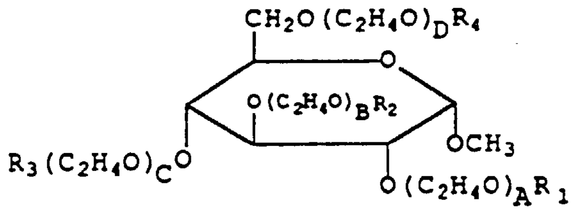

where

A, B, C and D = 21 - 75,
$R_1$, $R_2$, $R_3$ and $R_4$ = H or

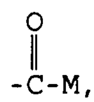

where

$R_1$-$R_4$ are not simultaneously H and
M = $C_{11}$-$C_{18}$-alkenyl or $C_{11}$-$C_{18}$-alkyl.

**2.** Fatty acid esters according to Claim 1, characterized in that
$R_1$ and $R_4$ =

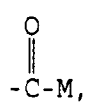

and $R_2$ and $R_3$ = H.

**3.** Fatty acid esters according to Claim 1, characterized in that
$R_1$ =

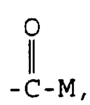

and $R_2$, $R_3$ and $R_4$ = H.

**4.** Fatty acid esters according to Claim 1, characterized in that
$R_4$ =

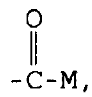

and $R_1$, $R_2$ and $R_3$ = H.

**5.** Fatty acid esters according to Claims 1 to 4, characterized in that M = $C_{17}$-alkenyl.

**6.** Process for the preparation of the fatty acid esters according to Claims 1 to 5, characterized in that

a) a methylglucoside is ethoxylated with 84 to 300 mol of ethylene oxide and
b) then esterified with 2 to 4 mol of a $C_{11}$ to $C_{18}$ saturated or unsaturated fatty acid in the absence of a solvent using an acid catalyst.

**7.** Process according to Claim 6, characterized in that the fatty acid used is oleic acid, stearic acid, linoleic acid, linolenic acid, lauric acid or a mixture thereof.

**8.** Process according to Claim 6 or 7, characterized in that the catalyst used is inorganic or organic acid.

**9.** Process according to Claim 8, characterized in that the catalyst used is sulphuric acid, hydrochloric acid, phosphoric acid, p-toluenesulphonic acid, methanesulphonic acid, ethanesulphonic acid or boron trifluoride.

**10.** Process according to Claims 6 to 9, characterized in that the reaction is carried out at temperatures between 110°C and 150°C without a solvent.

**11.** Use of the fatty acid esters according to Claims 1 to 5 as thickeners for surfactant-containing solutions in cosmetic preparations.

**Revendications**

**1.** Esters d'acides gras de dérivés de méthylglucoside de formule I

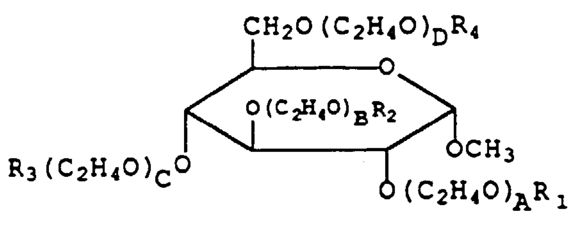

dans laquelle

A, B, C, D = 21-75
$R_1$, $R_2$, $R_3$, $R_4$ = H ou

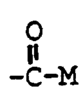 ,

$R_1$-$R_4$ n'étant pas simultanément H et
M = alcényle en $C_{11}$-$C_{18}$ ou alkyle en $C_{11}$-$C_{18}$.

**2.** Esters d'acides gras selon la revendication 1, caractérisés en ce que
$R_1$, $R_4$ =

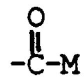

et $R_2$, $R_3$ = H.

**3.** Esters d'acides gras selon la revendication 1, caractérisés en ce que
$R_1$ =

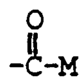

et $R_2$ , $R_3$ , $R_4$ = H.

**4.** Esters d'acides gras selon la revendication 1, caractérisés en ce que
$R_4$ =

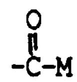

et $R_1$, $R_2$ , $R_3$ = H.

**5.** Esters d'acides gras selon les revendications 1 à 4, caractérisés en ce que M = alcényle en $C_{17}$.

**6.** Procédé de préparation de l'ester d'acide gras selon les revendications 1 à 5, caractérisé en ce que

a) un méthylglucoside est éthoxylé avec de 84 à 300 moles d'oxyde d'éthylène et
b) il est ensuite estérifié avec de 2 à 4 moles d'un acide gras saturé ou insaturé en $C_{11}$ à $C_{18}$, en l'absence de solvant, avec un catalyseur à action acide.

**7.** Procédé selon la revendication 6, caractérisé en ce que l'on utilise en tant qu'acide gras, l'acide oléique, l'acide stéarique, l'acide linoléique, l'acide linolénique, l'acide laurique ou un mélange de ceux-ci.

**8.** Procédé selon les revendications 6 ou 7, caractérisé en ce que l'on utilise en tant que catalyseur, des acides inorganiques ou organiques.

**9.** Procédé selon la revendication 8, caractérisé en ce que l'on utilise en tant que catalyseur, l'acide sulfurique, l'acide chlorhydrique, l'acide phosphorique, l'acide p-toluènesulfonique, l'acide méthanesulfonique, l'acide éthanesulfonique, le trifluorure de bore.

**10.** Procédé selon les revendications 6 à 9, caractérisé en ce que la réaction a lieu à des températures comprises entre 110°C et 150°C sans solvant.

**11.** Utilisation des esters d'acides gras selon la revendications 1 à 5, en tant qu'épaississants pour des solutions contenant un tensio-actif dans des formulations cosmétiques.